(19) Europäisches Patentamt
European Patent Office
Office européen des brevets

(11) **EP 3 620 274 A1**

(12) **EUROPEAN PATENT APPLICATION**
published in accordance with Art. 153(4) EPC

(43) Date of publication:
**11.03.2020 Bulletin 2020/11**

(51) Int Cl.:
**B25J 11/00** *(2006.01)*　　　**A61F 2/54** *(2006.01)*

(21) Application number: **18793956.6**

(22) Date of filing: **02.07.2018**

(86) International application number:
**PCT/IB2018/054890**

(87) International publication number:
**WO 2018/203318 (08.11.2018 Gazette 2018/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.05.2017   JP 2017091758**

(71) Applicant: **Innophys Co., Ltd.**
**Tokyo 162-0825 (JP)**

(72) Inventors:
• **KOBAYASHI, Hiroshi**
**Tokyo 162-8601 (JP)**

• **NAKAMURA, Eita**
**Tokyo 162-8601 (JP)**
• **ICHINOSE, Kouki**
**Tokyo 162-8601 (JP)**

(74) Representative: **Grünecker Patent- und Rechtsanwälte**
**PartG mbB**
**Leopoldstraße 4**
**80802 München (DE)**

Remarks:
The filing date of the international application is within two months from the date of expiration of the priority period (R. 26bis.3 PCT).

(54) **ARM AND LOWER BACK SUPPORT DEVICE**

(57)　An arm and lower back support device that is smaller in size, lighter and costs less. An arm and lower back support device (10) is provided with: an upper body frame (18) that is mounted on the back of a user; thigh arms (16) that are mounted on the lower limbs of the user; and arm support arms (20) that are mounted on the upper limbs of the user. In addition, the arm and lower back support device (10) comprises artificial muscles (22) which provide supportive force in the direction raising the upper body of the wearer, by urging the upper body frame (18) toward the thigh arms (16), and provide supportive force in the direction lifting the upper limbs of the user, by urging the arm support arms (20) toward the upper body frame (18).

FIG. 1

EP 3 620 274 A1

**Description**

TECHNICAL FIELD

The present invention relates to an arm and lower back support device.

BACKGROUND ART

Japanese Unexamined Patent Application, Publication No. 2009-273711 and Japanese Unexamined Patent Application, Publication No. 2016-2123 described below disclose arm and lower back support devices to support the lower back and an upper limb of a user by being mounted on the body of the user.

The arm and lower back support devices (upper arm holding device and upper arm support device) disclosed in Japanese Unexamined Patent Application, Publication No. 2009-273711 is configured to support lifting and holding of an upper arm of a user in response to actuation of a first actuator and to support the lower back of the user in response to actuation of a second actuator.

The arm and lower back support device (upper arm holding device and upper arm support device) disclosed in Japanese Unexamined Patent Application, Publication No. 2016-2123 is configured to support an upper limb of a user and to support the lower back of the user in response to actuation of a plurality of drive motors provided for corresponding joints of a human body.

DISCLOSURE OF THE INVENTION

Problems to be Solved by the Invention

Each of the arm and lower back support devices disclosed in Japanese Unexamined Patent Application, Publication No. 2009-273711 and Japanese Unexamined Patent Application, Publication No. 2016-2123 includes an actuator for generating supportive force for supporting an upper limb of a user and an actuator for generating supportive force for supporting the lower back of the user that are provided independently of each other. This increases the number of constituent parts, so that a room is left for improvement in terms of size reduction, weight reduction, and cost reduction of an arm and lower back support device.

In view of the foregoing fact, the present invention is intended to provide an arm and lower back support device capable of encouraging size reduction, weight reduction, and cost reduction.

Means for Solving the Problems

An arm and lower back support device of a first aspect comprises: a back mount to be mounted on the back of a user; a lower limb mount to be mounted on a lower limb of the user; an upper limb mount to be mounted on an upper limb of the user; and a supportive force provider that provides supportive force acting in a direction of raising the upper body of the user by urging the back mount toward the lower limb mount, and provides supportive force acting in a direction of lifting the upper limb of the user by urging the upper limb mount toward the back mount.

According to the arm and lower back support device of the first aspect, the back mount is mounted on the back of the user, the lower limb mount is mounted on the lower limb of the user, and the upper limb mount is mounted on the upper limb of the user, thereby mounting the arm and lower back support device on the body of the user. The supportive force provider provides the supportive force acting in the direction of raising the upper body of the user and the supportive force acting in the direction of lifting the upper limb of the user by urging the back mount toward the lower limb mount and urging the upper limb mount toward the back mount. According to the invention of the first aspect, the supportive force provider generates the two supportive forces including the supportive force acting in the direction of raising the upper body of the user and the supportive force acting in the direction of lifting the upper limb of the user. This makes it possible to encourage size reduction, weight reduction, and cost reduction of the arm and lower back support device, compared to a case where actuators are provided independently of each other for generating two supportive forces including supportive force acting in a direction of raising the upper body of a user and supportive force acting in a direction of lifting an upper limb of the user.

According to an arm and lower back support device of a second aspect, in the arm and lower back support device of the first aspect, the supportive force provider is an artificial muscle to contract in response to supply of gas, and the supportive force acting in the direction of raising the upper body of the user and the supportive force acting in the direction of lifting the upper limb of the user are provided by the contraction of the artificial muscle.

According to the arm and lower back support device of the second aspect, the artificial muscle functions as the supportive force provider for generating the supportive forces. This makes it possible to generate larger supportive force while suppressing weight increase of the supportive force provider.

According to an arm and lower back support device of a third aspect, in the arm and lower back support device of the first aspect, the supportive force provider is a spring, and the supportive force acting in the direction of raising the upper body of the user and the supportive force acting in the direction of lifting the upper limb of the user are provided by the deformation of the spring caused by change in the posture of the user.

According to the arm and lower back support device of the third aspect, the spring functions as the supportive force provider for generating the supportive forces. The supportive forces are generated by the deformation of the spring. This configuration allows generation of the supportive forces in the absence of electrical or mechanical energy to be supplied from outside.

According to an arm and lower back support device of a fourth aspect, in the arm and lower back support device of any one of the first to third aspects, the upper limb mount is tiltable relative to the back mount in a right-left direction viewed from the user.

According to the arm and lower back support device of the fourth aspect, the upper limb mount is tiltable relative to the back mount in the right-left direction viewed from the user. This allows the user to be responsive to packages of various sizes in the operation of lifting the packages, etc.

According to an arm and lower back support device of a fifth aspect, in the arm and lower back support device of any one of the first to fourth aspects, the upper limb mount includes a wrist mount to be mounted on a wrist of the user, and supportive force acting in a direction of lifting the upper limb of the user is provided from the wrist mount to the wrist of the user.

According to the arm and lower back support device of the fifth aspect, when the user lifts a package or keeps a state of lifting the package, the wrist to be subjected to load can be supported effectively by the wrist mount.

According to an arm and lower back support device of a sixth aspect, in the arm and lower back support device of any one of the first to fifth aspects, the upper limb mount is supported in a tiltable manner on the back mount through an upper limb mount side pulley, the lower limb mount is supported in a tiltable manner on the back mount through a lower limb mount side pulley, the upper limb mount side pulley and the lower limb mount side pulley have different outer diameters, and a wire connected to the supportive force provider is engaged with each of the upper limb mount side pulley and the lower limb mount side pulley.

According to the arm and lower back support device of the sixth aspect, the wire connected to the supportive force provider is engaged with each of the upper limb mount side pulley and the lower limb mount side pulley having different outer diameters. This makes it possible to generate a difference between a value of displacement of the upper body mount relative to the lower limb mount and a value of displacement of the upper limb mount relative to the upper body mount. More specifically, according to the invention of the sixth aspect, optimization of a ratio between a value of displacement of the upper body mount relative to the lower limb mount and a value of displacement of the upper limb mount relative to the upper body mount can be encouraged further in response to a way in which the user conducts work.

An arm and lower back support device of a seventh aspect comprises: a back mount to be mounted on the back of a user; a lower limb mount to be mounted on a lower limb of the user; an upper limb mount to be mounted on an upper limb of the user; and a supportive force provider that provides supportive force acting in a direction of raising the upper body of the user by urging the back mount toward the lower limb mount, and provides supportive force acting in a direction of lifting the upper limb of the user by urging the upper limb mount toward the back mount. A ratio of distribution between the supportive force acting in the direction of lifting the upper limb of the user and the supportive force acting in the direction of raising the upper body of the user changes in response to motion of the user.

According to the arm and lower back support device of the seventh aspect, a ratio of distribution between the supportive force acting in the direction of lifting the upper limb of the user and the supportive force acting in the direction of raising the upper body of the user can be changed in response to motion of the user, namely, in response to change in the posture of the user.

Effects of the Invention

The arm and lower back support device according to the present invention has the excellent effect of capable of encouraging size reduction, weight reduction, and cost reduction.

BRIEF DESCRIPTION OF THE DRAWINGS

Fig. 1 is a perspective view showing an arm and lower back support device according to a first embodiment;

Fig. 2 is a side view showing the arm and lower back support device according to the first embodiment;

Fig. 3 is a front view showing the arm and lower back support device according to the first embodiment;

Fig. 4 is a plan view showing the arm and lower back support device according to the first embodiment;

Fig. 5 is a side view showing a supporter;

Fig. 6 is a schematic view schematically showing an artificial muscle;

Fig. 7 is a schematic view schematically showing a state in which air is supplied to the artificial muscle shown in Fig. 6 and corresponding to Fig. 5;

Fig. 8 is a schematic view schematically showing the artificial muscle arranged in an artificial muscle housing;

Fig. 9 is a side view schematically showing principal parts of the arm and lower back support device in a state before air is supplied;

Fig. 10 is a side view schematically showing the principal parts of the arm and lower back support device in a state after air is supplied;

Fig. 11 is a perspective view for explaining each tilting axis of the arm and lower back support device corresponding to Fig. 1;

Fig. 12 is an explanatory view for explaining a method of evaluating the amount of muscle usage;

Fig. 13A is a side view showing a state in which a user wearing the arm and lower back support device starts to lift a package from a posture of not dropping a lower back;

Fig. 13B is a side view showing a state in which the user wearing the arm and lower back support device in the posture shown in Fig. 13A has finished lifting the package;

Fig. 13C is a side view showing a state in which the user wearing the arm and lower back support device holds the package while the user is in a posture of lifting the package;

Fig. 14A is a side view showing a state in which a user wearing the arm and lower back support device starts to lift a package from a posture of dropping a lower back;

Fig. 14B is a side view showing a state in which the user wearing the arm and lower back support device in the posture shown in Fig. 14A has finished lifting the package;

Fig. 14C is a side view showing a state in which the user wearing the arm and lower back support device holds the package while the user is in a posture of lifting the package;

Fig. 15 is an explanatory view for explaining points of the body of a user where myoelectric potentials were measured;

Fig. 16 is a graph showing the amount of muscle usage of each subject used from the state shown in Fig. 13A to the state shown in Fig. 13B;

Fig. 17 is a graph showing the amount of muscle usage of each subject used while the subject lifts the package in the state shown in Fig. 13C;

Fig. 18 is a graph showing the amount of muscle usage of each subject used from the state shown in Fig. 14A to the state shown in Fig. 14B;

Fig. 19 is a graph showing the amount of muscle usage of each subject used while the subject lifts the package in the state shown in Fig. 14C;

Fig. 20 is a side view showing a state in which a user is wearing an arm and lower back support device according to a second embodiment;

Fig. 21 is an explanatory view for explaining an air spring; Fig. 22 is a side view showing a state in which a user wearing the arm and lower back support device according to the second embodiment drops a lower back;

Fig. 23 is a side view schematically showing principal parts of the arm and lower back support device according to the second embodiment;

Fig. 24 is a side view showing a state in which a user is wearing the arm and lower back support device according to the second embodiment;

Fig. 25 is a side view showing a state in which a user wearing the arm and lower back support device according to the second embodiment takes hold of a package in a posture of not dropping a lower back;

Fig. 26 is a side view showing a state in which the user wearing the arm and lower back support device according to the second embodiment and holding the package drops the lower back;

Fig. 27 is a side view showing a state in which the user wearing the arm and lower back support device according to the second embodiment and holding the package lifts the lower back from the state shown in Fig. 26;

Fig. 28A is a side view schematically showing principal parts of the arm and lower back support device in the state shown in Fig. 25;

Fig. 28B is a side view schematically showing the principal parts of the arm and lower back support device in the state shown in Fig. 26;

Fig. 28C is a side view schematically showing the principal parts of the arm and lower back support device in the state shown in Fig. 27;

Fig. 29 is a perspective view showing an arm and lower back support device according to a third embodiment;

Fig. 30 is a side view showing the arm and lower back support device according to the third embodiment;

Fig. 31 is a front view showing the arm and lower back support device according to the third embodiment; and

Fig. 32 is a plan view showing the arm and lower back support device according to the third embodiment.

PREFERRED MODE FOR CARRYING OUT THE INVENTION

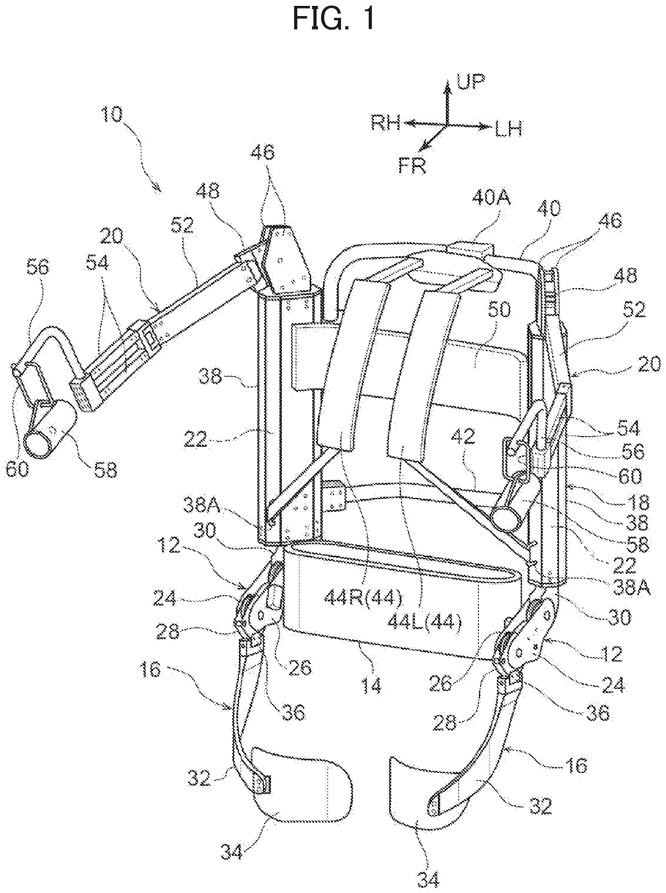

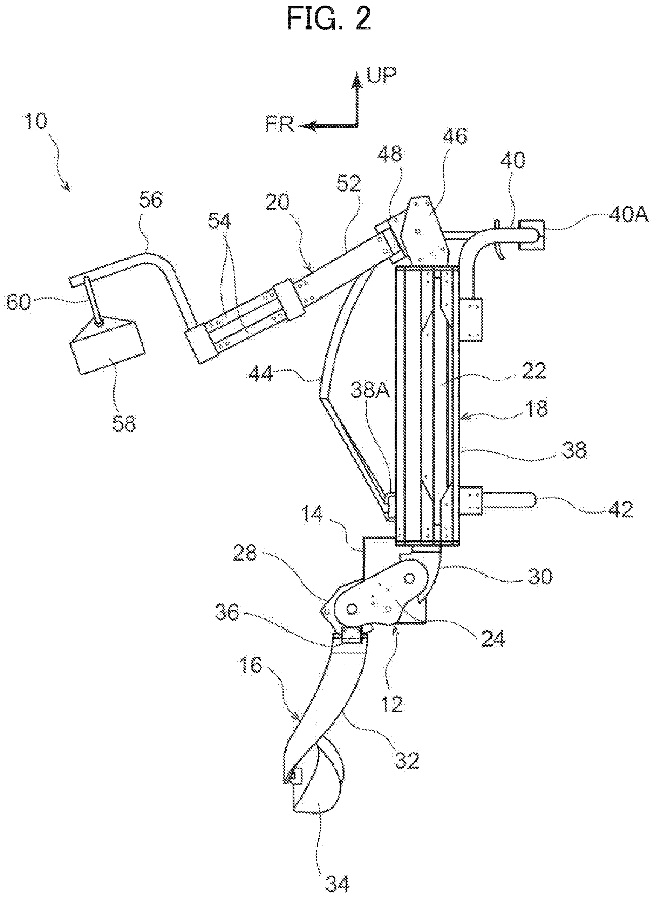

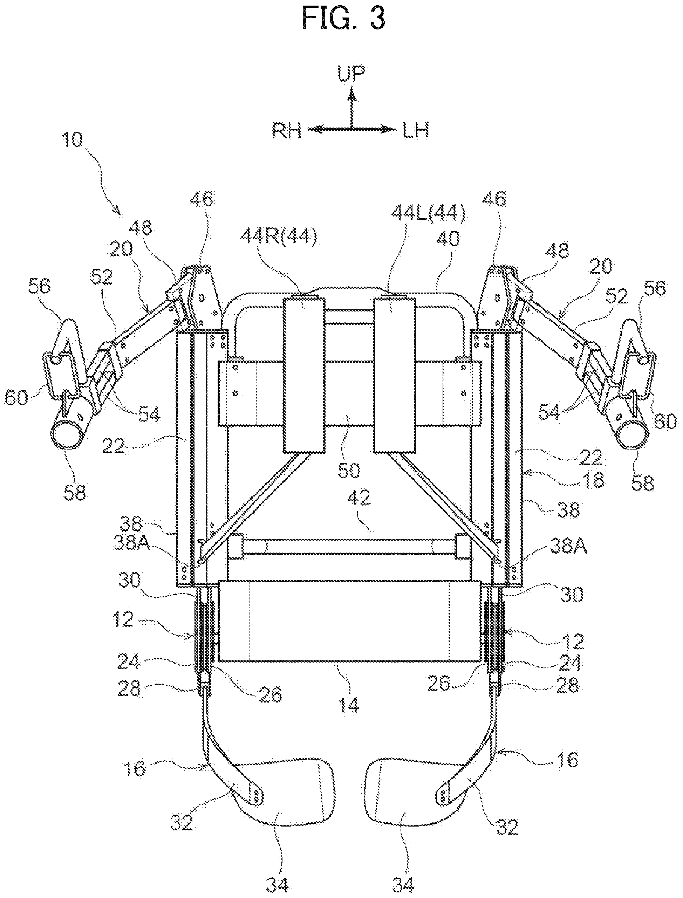

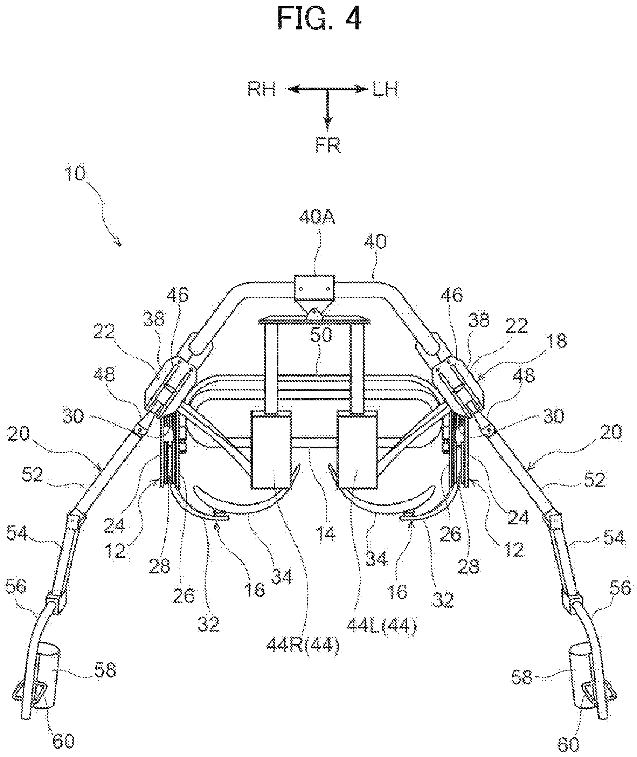

An arm and lower back support device according to a first embodiment of the present invention will be described using Figs. 1 to 10. An arrow FR shows a front side of a front-back direction, an arrow RH and an arrow LH show a right side and a left side respectively, and an arrow UP shows an upper side of an up-down direction viewed from a user wearing the arm and lower back support device and in an upright posture. In the following description, where the front-back direction, right-left direction, and up-down direction are simply referred to, these directions mean the front-back direction, right-left direction, and up-down direction respectively as viewed from a user wearing the arm and lower back support device and in an upright posture.

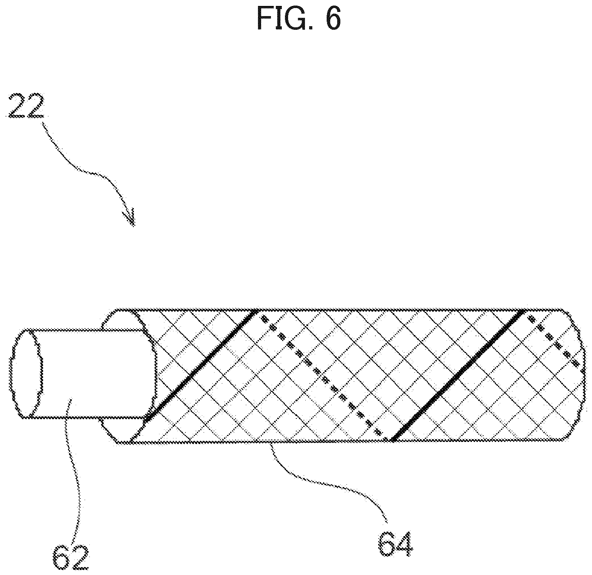

As shown in Figs. 1 to 4, an arm and lower back support device 10 includes right and left base units 12 in a pair arranged lateral to the lower back of a user, and a lower back belt 14 attached to the base units 12 and to be mounted on the lower back of the user. The arm and lower back support device 10 includes right and left thigh arms 16 in a pair as a lower limb mount arranged along the thighs of the user and attached in a tiltable manner to the base units 12. The arm and lower back support device 10 further includes an upper body frame 18 arranged along the upper body (back) of the user, attached in a tiltable manner to the base units 12, and forming a part of a back mount. The arm and lower back support device 10 also includes an arm support arm 20 as an upper limb mount arranged along an upper limb of the user and attached in a tiltable manner to an upper end portion of the upper body frame 18. The arm and lower back support device 10 further includes an artificial muscle 22 (see Fig. 6) as a supportive force provider arranged in the upper body frame 18.

The base unit 12 has a configuration including an outer plate 24 and an inner plate 26 each formed into a plate-like shape. The outer plate 24 and the inner plate 26 are formed into the approximately same shape in a side view (as viewed from the right side or left side). The outer plate 24 and the inner plate 26 are connected through a plurality of connection pins, for example. By doing so, the outer plate 24 and the inner plate 26 are spaced by a predetermined interval and arranged parallel to each other in the right-left direction.

A first pulley 28 and a second pulley 30 are provided in a rotatable manner between the outer plate 24 and the inner plate 26. The first pulley 28 as a lower limb mount side pulley is attached to lower portions of the outer plate 24 and the inner plate 26 while being configured to rotate about an axis extending in a direction corresponding to the right-left direction. The rotary axis of the first pulley 28 corresponds to the hip joint of a user. The thigh arm 16 described later is attached to the first pulley 28. As the first pulley 28 is rotated toward one side (rotated anticlockwise in a left side view), the first pulley 28 abuts on an abutment target fixed to the outer plate 24 and the inner plate 26, for example. By doing so, the rotation of the first pulley 28 toward the one side is regulated. As a result, tilt of the thigh arm 16 attached to the first pulley 28 toward the back side is limited.

The second pulley 30 is attached to upper portions of the outer plate 24 and the inner plate 26 and on the back side of the first pulley 28 while being configured to rotate about an axis extending in a direction corresponding to the right-left direction. The rotary axis of the second pulley 30 corresponds to the sacroiliac joint of a user. The upper body frame 18 described later is attached to the second pulley 30.

The lower back belt 14 is formed into a ring-like shape as viewed from the upper side of a user. The lower back belt 14 is to be mounted on the lower back of the user. The lower back belt 14 is provided with a length adjuster not shown for adjusting the peripheral length of the lower back belt 14.

The thigh arm 16 has a configuration including an arm body 32 extending along a thigh of a user, and a thigh pad 34 attached to the arm body 32. The arm body 32 is formed by bending a plate-like member, for example. The arm body 32 is attached to the first pulley 28 through a hinge 36. By doing so, the thigh arm 16 is attached to the base unit 12 in such a manner as to be capable of tilting in the front-back direction. In the first embodiment, the hinge 36 is configured to be capable of pivoting in the right-left direction to further allow the thigh arm 16 to tilt in the right-left direction relative to the base unit 12.

The thigh pad 34 has a curved shape conforming to a surface of a thigh of a user on the front side. The thigh pad 34 is attached to a lower end portion of the arm body 32. The thigh pad 34 is configured to be capable of pivoting to a predetermined angle relative to the arm body 32 about an axis extending in a direction corresponding to the right-left direction. This makes it possible to maintain a state of contact between the thigh pad 34 and the surface of the thigh of the user on the front side at an intended contact state.

The upper body frame 18 is formed into an approximately ladder-like shape in a front view (as viewed from the front side of a user). The upper body frame 18 includes right and left artificial muscle attachments 38 in a pair spaced by a predetermined interval in the right-left direction and storing artificial muscles 22 therein (see Fig. 6), an upper connection 40 connecting upper portions of the right and left artificial muscle attachments 38 in a pair in the right-left direction, and a lower connection 42 connecting lower portions of the right and left artificial muscle attachments 38 in a pair in the right-left direction. Respective lower end portions of the right and left artificial muscle attachments 38 in a pair and a central portion of the upper connection 40 defined in the right-left direction are provided with upper body mount belt attachments 38A and an upper body mount belt attachment 40A respectively to which an upper body mount belt 44 to be mounted on the upper body of a user is attached. The upper body mount belt 44 forming the other part of the back mount has a configuration including a right side mount belt 44R to be mounted on the right shoulder of the user, and a left side mount belt 44L to be mounted on the left shoulder of the user. The respective lengths of the right side mount belt 44R and the left side mount belt 44L are adjustable.

The artificial muscle attachments 38 in a pair each have a lower end portion fixed to the second pulley 30. This allows the upper body frame 18 to tilt in the front-back direction relative to the base unit 12. The artificial muscle attachments 38 in a pair each have an upper end portion to which pulley support plates 46 in a pair parallel to each other are fixed. A third pulley 48 is provided between the pulley support plates 46 in a pair. The third pulley 48 is an upper limb mount side pulley to which the arm support arm 20 described later is attached. The third pulley 48 is rotatably supported by the pulley support plates 46 in a pair.

A back pad 50 is stretched between intermediate positions of the artificial muscle attachments 38 in a pair defined in the up-down direction.

The arm support arm 20 has a configuration including a first arm part 52, a second arm part 54, and a tip arm part 56.

The first arm part 52 is formed into a rectangular columnar shape. The first arm part 52 has a one-side end portion supported on the third pulley 48. By doing so, the arm support arm 20 is supported in a tiltable manner on an upper end portion of the artificial muscle attachment 38 of the upper body frame 18. The first arm part 52 and the third pulley 48 are joined with a pin. This allows the first arm part 52 to tilt in the right-left direction relative to the third pulley 48.

The second arm part 54 is formed using two rectangular columnar members thinner than the first arm part 52. The second arm part 54 has a one-side end portion joined to the other-side end portion of the first arm part 52. The second arm part 54 and the first arm part 52 are joined with pins. This allows the second arm part 54 to tilt in the right-left direction relative to the first arm part 52.

The tip arm part 56 is formed by bending a round rod-like material into an L shape, for example. The tip arm part 56 has a one-side end portion fixed to the other-side end portion of the second arm part 54. The tip arm part 56 has the other-side end portion to which a supporter 58 is attached through a supporter engagement wire 60. The supporter 58 is a wrist mount into which a hand of a user is to be inserted.

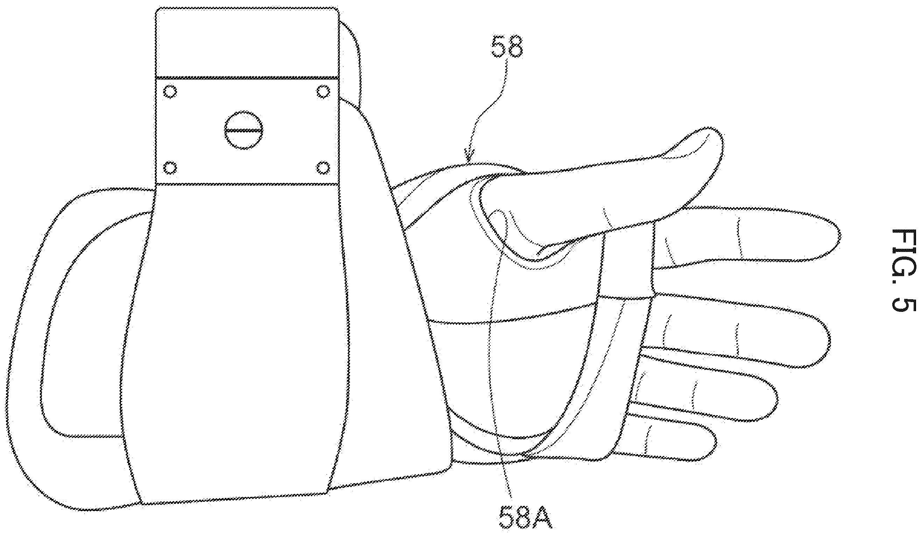

As shown in Fig. 5, the supporter 58 is made of a material having stretching properties. The supporter 58 is formed into a cylindrical shape covering an area from the wrist to the palm and back of a hand of a user. The supporter 58 is provided with a thumb insertion hole 58A for letting the thumb of the hand pass through. A section from the index finger to the little finger of the hand is to project from the tip of the supporter 58.

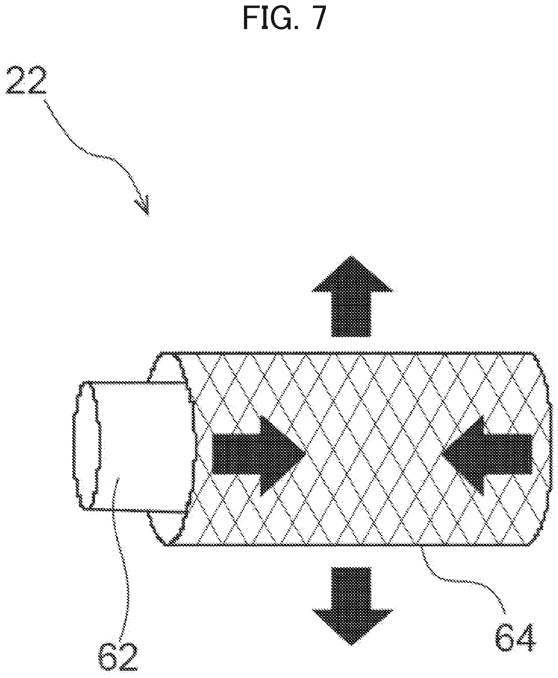

As shown in Figs. 6 and 7, the artificial muscle 22 is what is called a McKibben-type artificial muscle. The artificial muscle 22 has a configuration including an elastic tube 62 formed into a tubular shape using an elastic material such as rubber, and a cylindrical mesh sleeve 64 covering the elastic tube 62. The artificial muscle 22 is formed by covering the elastic tube 62 with the mesh sleeve 64 and being swaged at the opposite ends.

The elastic tube 62 has space therein to be supplied with gas (air). Air is to be supplied into the elastic tube 62 through a tank not shown storing compressed air and a connection pipe.

The mesh sleeve 64 is finished by being woven with a wire rod such as high-tensile fiber having low stretching properties, for example. When air is supplied into the elastic tube 62 arranged in the mesh sleeve 64 to expand the elastic tube 62, the size of the mesh sleeve 64 is increased in a direction perpendicular to the direction of the length of the mesh sleeve 64 and the size of the mesh sleeve 64 is reduced in the direction of the length of the mesh sleeve 64, as shown in Fig. 7. This shortens the length of the artificial muscle 22 (contracts the artificial muscle 22) to generate force of supporting a lower back and an arm described later. Supplying gas (compressed air) to the inner elastic tube 62 allows generation of large contractile force of up to 2000 N in the lengthwise direction of the artificial muscle 22. The artificial muscle 22 has a low weight of about 130 g, and has a considerably larger mass to power ratio than motors, etc.

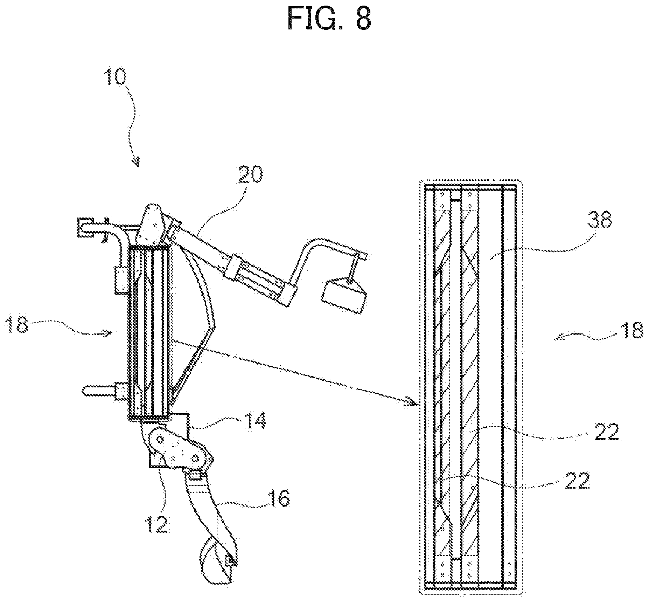

As shown in Fig. 8, in the first embodiment, the foregoing artificial muscle 22 includes two artificial muscles 22 provided inside the right-side artificial muscle attachment 38 and two artificial muscles 22 provided inside the left-side artificial muscle attachment 38. The elastic tubes 62 of the artificial muscles 22 on the right side and those on the left side arranged inside the corresponding artificial muscle attachments 38 are configured to be supplied with air simultaneously.

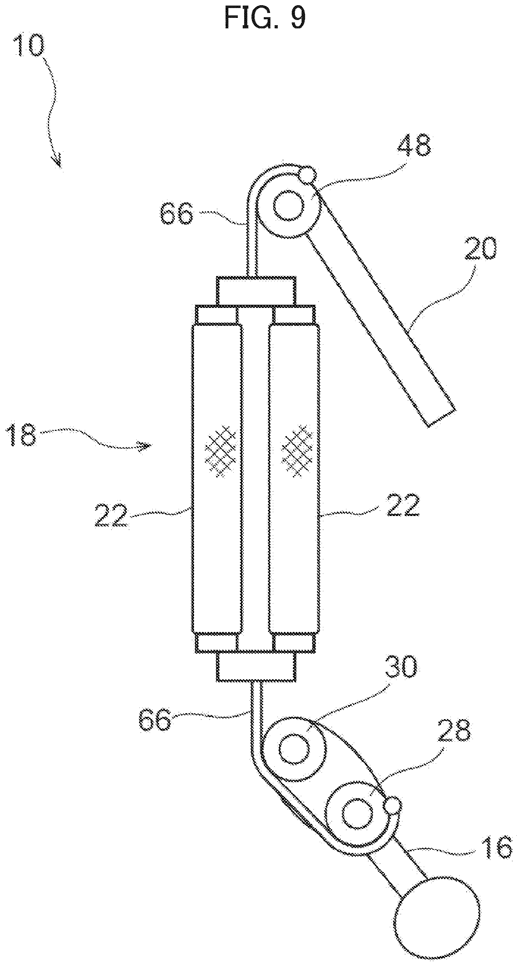

As schematically shown in Fig. 9, respective one-side end portions of the two artificial muscles 22 arranged inside the right-side artificial muscle attachment 38 are engaged with the right-side third pulley 48 through a wire 66, and the respective other-side end portions of these two artificial muscles 22 are engaged with the right-side first pulley 28 through the wire 66. Likewise, respective one-side end portions of the two artificial muscles 22 arranged inside the left-side artificial muscle attachment 38 are engaged with the left-side third pulley 48 through the wire 66, and the respective other-side end portions of these two artificial muscles 22 are engaged with the left-side first pulley 28 through the wire 66. In the first embodiment, the first pulley 28 and the third pulley 48 each have an outer diameter (an outer diameter at a section with the wound wire 66) of 50 mm.

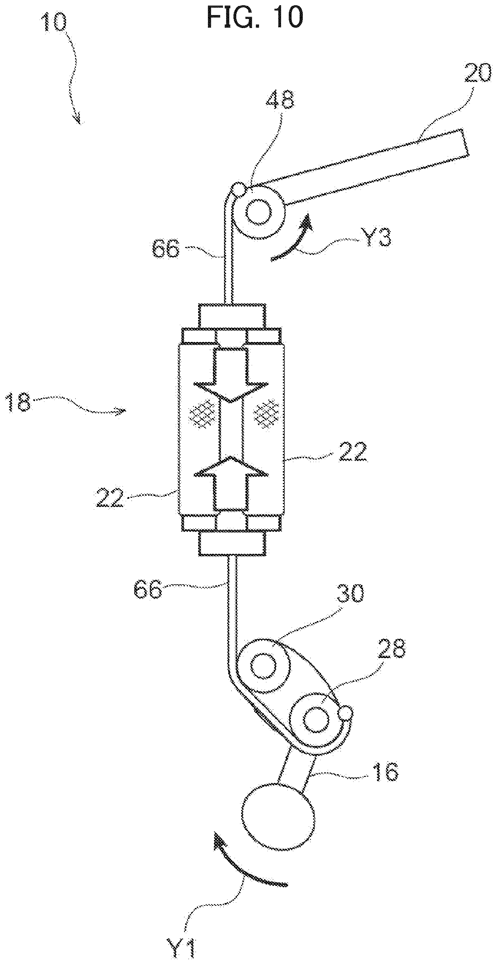

As shown in Fig. 10, by the contraction of the artificial muscle 22, the thigh arm 16 is tilted toward the back side (urged in a direction of being tilted) and the arm support arm 20 is tilted toward the upper side (urged in a direction of being tilted).

(Operation and effect of first embodiment)

The operation and effect of the first embodiment will be described next.

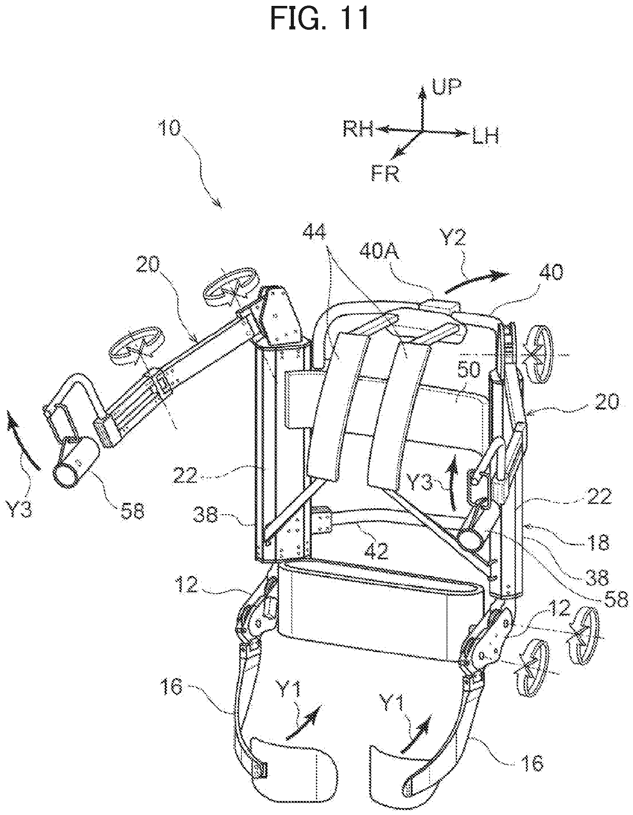

As shown in Fig. 11, according to the foregoing arm and lower back support device 10 of the first embodiment, the lower back belt 14, the upper body mount belt 44, and the thigh arms 16 are mounted on the lower back, upper body, and thighs of a user respectively. Further, the supporters 58 are mounted on the both hands of the user, thereby mounting the arm and lower back support device 10 on the body of the user. In the first embodiment, the provision of the back pad 50 prevents the back surface of the user from contacting the upper body mount belt attachment 40A and the artificial muscle attachments 38 in a pair. To ensure a sufficient shoulder width during mounting of the arm and lower back support device 10, the shoulder widths of several persons were measured and the shoulder width of the device was set at 500 mm. The arm and lower back support device 10 of the first embodiment can be mounted in about 25 seconds.

As shown in Figs. 10 and 11, when gas is supplied to the artificial muscles 22 in response to user' operation on a switch, for example, the artificial muscles 22 contract. This urges the thigh arms 16 in directions of being tilted toward the back side (urges the thigh arms 16 in the directions of arrows Y1), while generating reaction of urging the upper body frame 18 in a direction of raising the upper body of the user (inputting supportive force acting in the direction of an arrow Y2 to the lower back of the user). As the artificial muscles 22 contract, the arm support arms 20 are urged in directions of being tilted toward the upper side. By doing so, supportive forces of lifting the hands of the user (directions of arrows Y3) are input to the hands (wrists) of the user. As a result, the arm and lower back support device 10 of the first embodiment makes it possible to reduce load to be applied to the lower back and arms (upper limbs) of the user when the user wearing the arm and lower back support device 10 lifts a package or keeps a state of lifting the package.

The development of mechanical technology has produced transport equipment and industrial robots and has automated dangerous and simple works, so that people are in the process of being released from extreme physical fatigue. In a wide range of fields including manufacturing industries, transport service, and caregiving service, however, many heavy works difficult to mechanize are still left. A survey conducted by European Agency for Safety and Health at Work (EU-OSHA) shows that millions of people are developing musculoskeletal disorders (MSDs) every year only in European countries due to hard physical labor or lifting of packages, etc., 33% of laborers as a whole are required to handle heavy items, and 17% of those laborers complain of sores on their arms or feet. The MSDs have also caused serious economic loss. In France in 2006, this loss exceeds about 700 million Euro and loss caused by the MSDs on upper limbs amounts to 2% of the GNP. As a countermeasure against these problems, a wearable muscle support unit, a muscle suit, has been developed for assisting in the motion of a human physically using an exoskeleton. A physical laborer is subjected not only to load applied to a lower back but also to heavy load applied to a shoulder or an upper limb such as an upper arm. In this regard, the arm and lower back support device 10 of the first embodiment is used effectively with the intention of reducing load on upper limbs and a lower back to be applied during motion of lifting a package.

In the first embodiment, the artificial muscle 22 generates two supportive forces including supportive force acting in a direction of raising the upper body of a user and supportive force acting in a direction of lifting an arm of the user. This makes it possible to encourage size reduction, weight reduction, and cost reduction of the arm and lower back support device 10, compared to a case where actuators are provided independently of each other for generating two supportive forces including supportive force acting in a direction of raising the upper body of a user and supportive force acting in a direction of lifting an arm of the user.

In the first embodiment, the artificial muscle 22 functions as a member for generating the foregoing supportive forces. This makes it possible to generate larger supportive force while suppressing weight increase of the member for generating the foregoing supportive forces.

In the first embodiment, the arm support arm 20 has a configuration including the first arm part 52 and the second arm part 54. The first arm part 52 is tiltable in the right-left direction relative to the third pulley 48, and the second arm part 54 is tiltable in the right-left direction relative to the first arm part 52. This allows a user to be responsive to packages of various sizes in the operation of lifting the packages, etc.

In the first embodiment, when a user lifts a package or keeps a state of lifting the package, a wrist to be subjected to load can be supported effectively by the supporter 58.

(Quantitative evaluation of effect of support by arm and lower back support device 10)

The following describes a result of quantitative evaluation about the effect of support achieved by the arm and lower back support device 10.

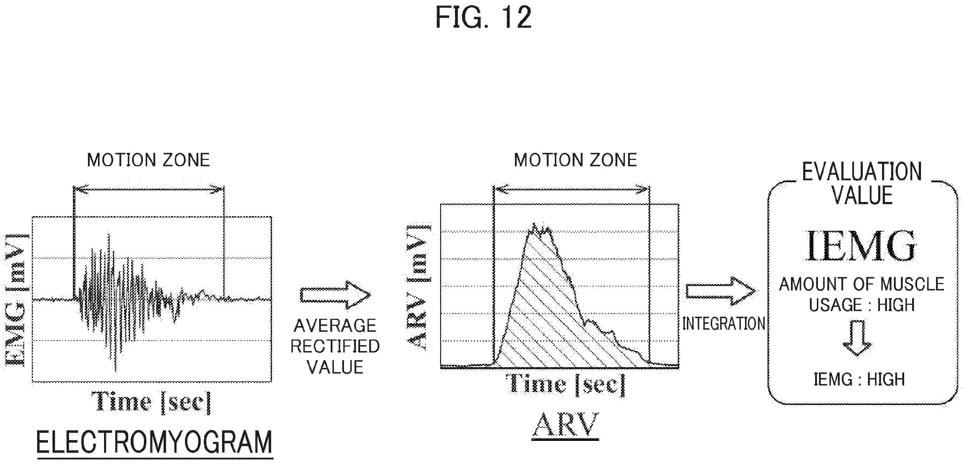

As shown in Fig. 12, to evaluate the effect of support achieved by the arm and lower back support device 10, a surface electromyogram (hereinafter called "EMG") is measured. For the measurement, WEB-7000 available from Nihon Kohden Corporation was used at a sample frequency of 250 Hz and a time constant of 0.1 sec. Cutoff frequencies were 30 Hz for a low-pass filter and 500 Hz for a high-pass filter. The measured EMG is subjected to average rectified value analysis ("hereinafter called "ARV analysis"). A resultant myoelectric potential is integrated and a resultant integral electromyogram (hereinafter called "IEMG") is evaluated as the amount of muscle usage. By using $\tau$ meaning a time constant, (-T, T) meaning a calculation zone, and t meaning time for the ARV analysis, the IEMG can be calculated from the following mathematical formulas 1 and 2:

[Math. 1]

$$\mathrm{ARV}(t) = \frac{1}{2T}\int_{-\tau}^{T}|S(t+\tau)|d\tau$$

[Math. 2]

$$\mathrm{IEMG}(t) = \sum \mathrm{ARV}(t)\varDelta t$$

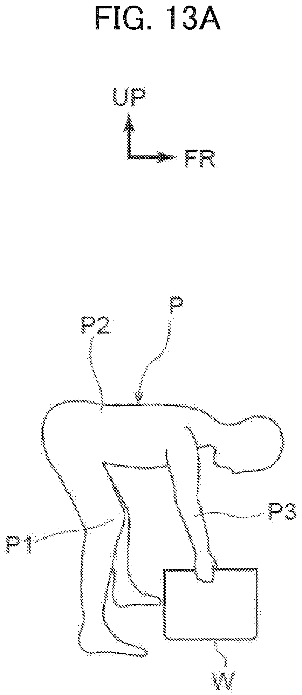

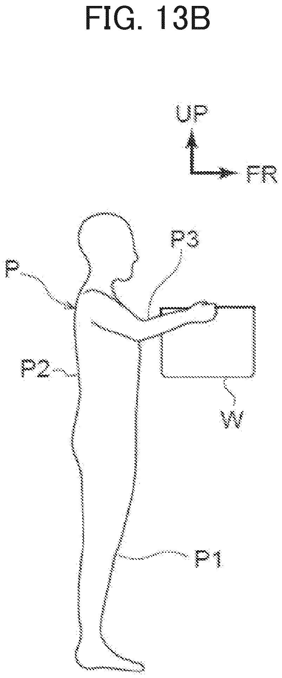

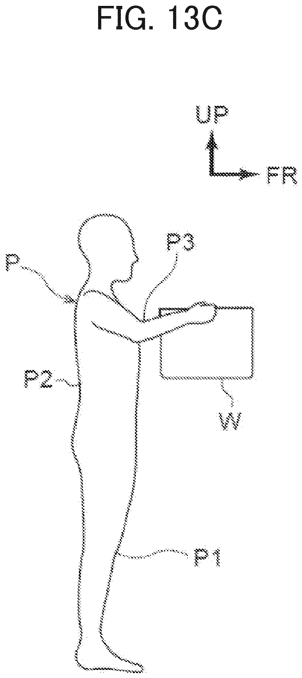

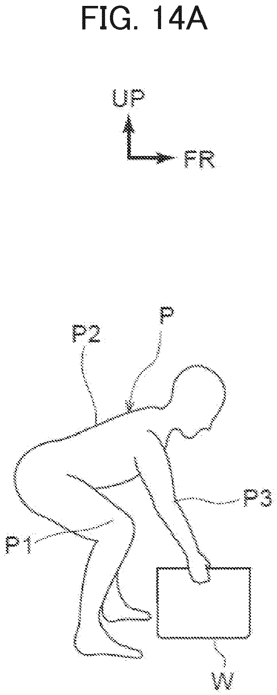

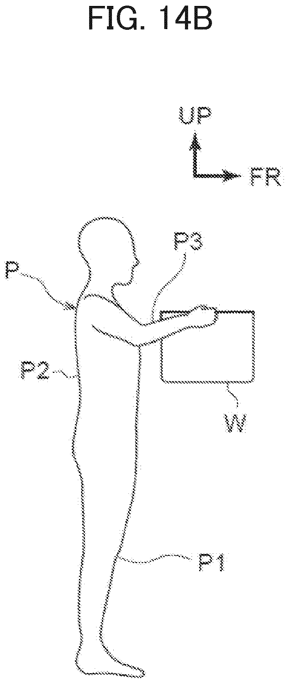

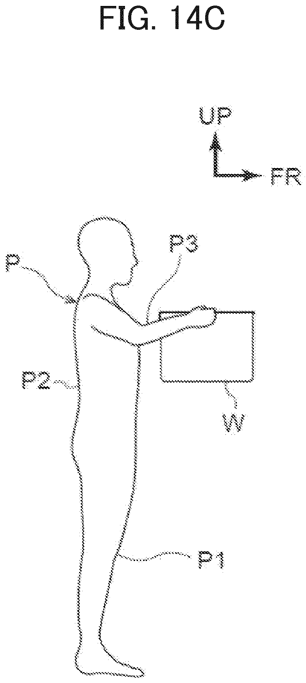

Subjects (users P) were healthy three men in their twenties. As shown in Figs. 13A, 13B, 14A, and 14B, a zone of three seconds taken in the motion of lifting a package W weighing 10 kg placed on a floor is called a zone 1. As shown in Figs. 13B, 13C, 14B, and 14C, a zone of three seconds taken in the motion of holding the package W is called a zone 2. Then, the IEMG in a state of not wearing the arm and lower back support device 10 and the IEMG in a state of wearing the arm and lower back support device 10 are compared. The package W was lifted in two ways: a way of lifting with a lower back in a raised position as shown in Fig. 13A (hereinafter called a lifting way X); and a way of lifting with a lower back in a dropped position as shown in Fig. 14A (hereinafter called a lifting way Y). Compressed air was poured into the artificial muscle 22 using a valve for direct switching of air.

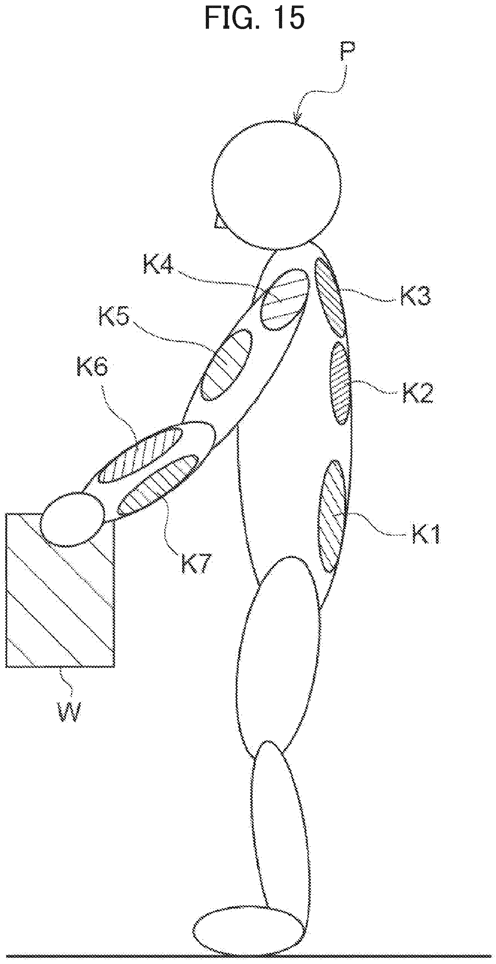

As shown in Fig. 15, there are seven muscles to be measured including an erector muscle of spine K1, a broadest muscle of back K2, a cowl muscle K3, an anterior deltoid K4, a biceps muscle of arm K5, a flexor carpi radialis muscle K6, and a flexor carpi ulnaris muscle K7.

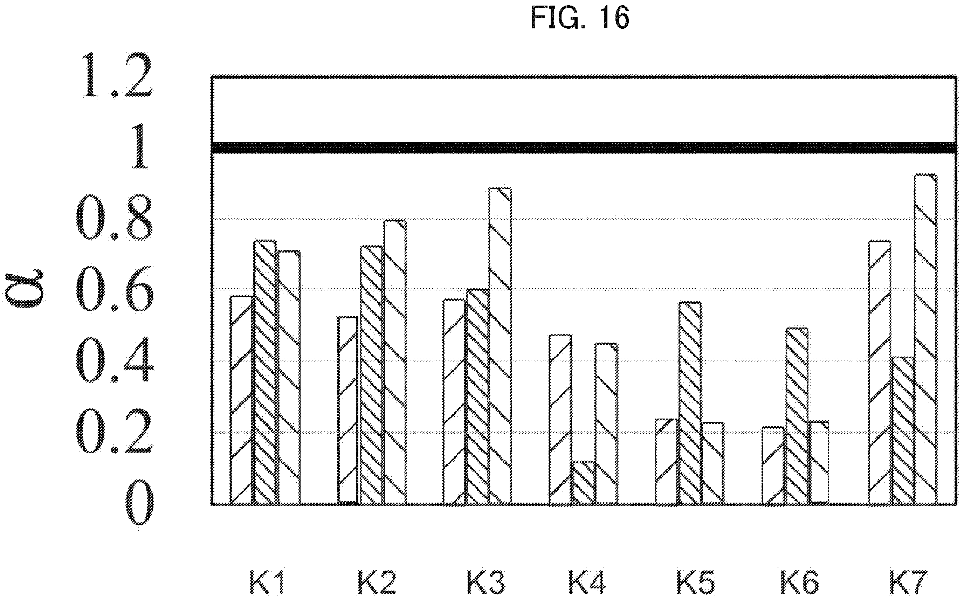

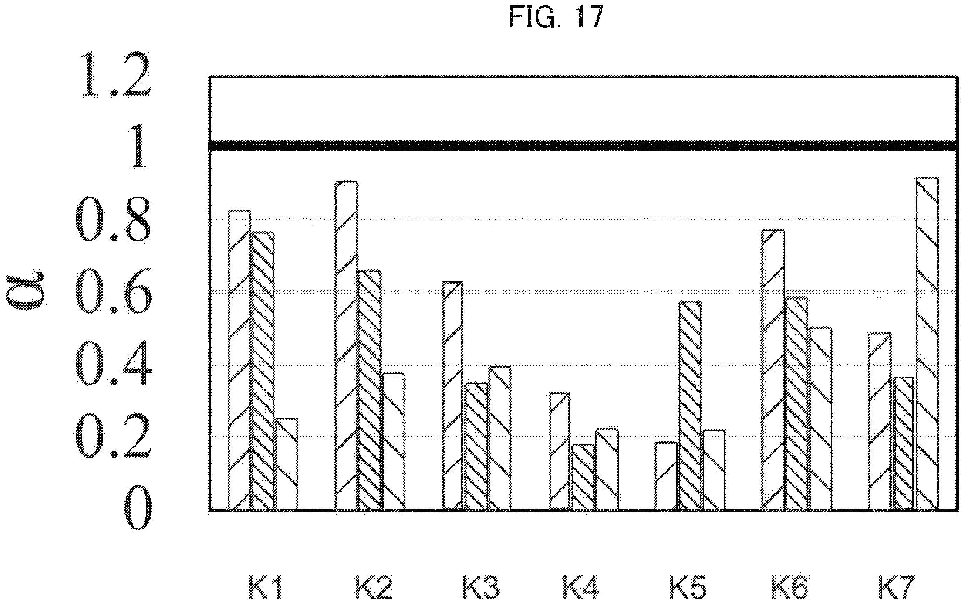

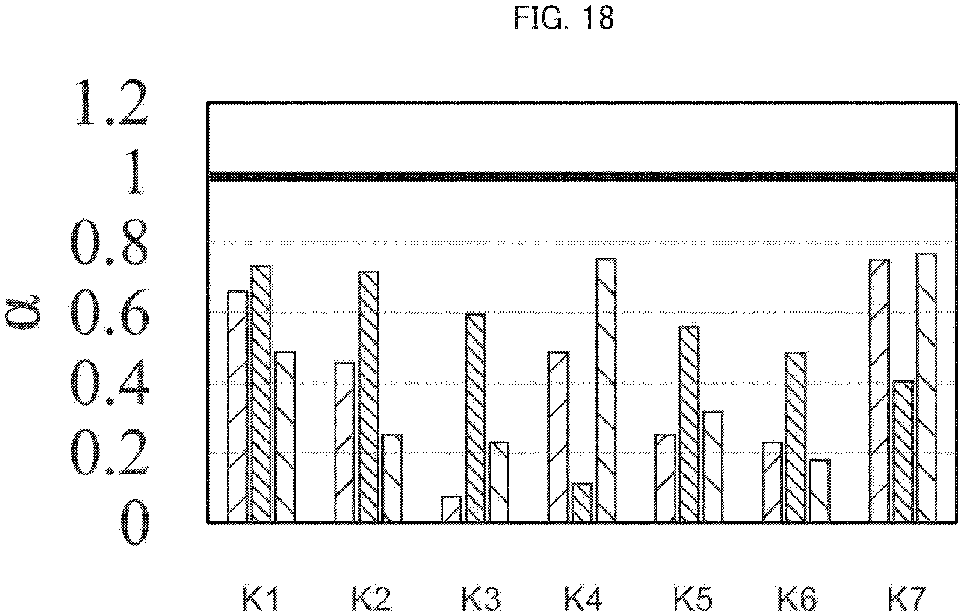

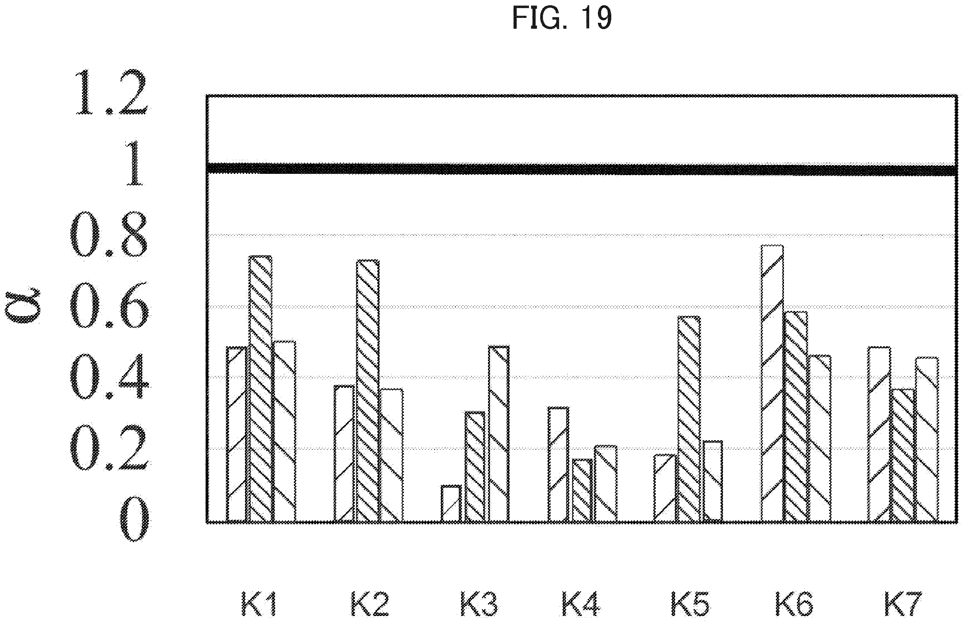

Regarding the three subjects subjected to the measurement, Fig. 16 shows relative IEMG values in a wearing state relative to IEMG values set at 1 in a non-wearing state determined in the zone 1 using the lifting way X, Fig. 17 shows such relative IEMG values determined in the zone 2 using the lifting way X, Fig. 18 shows such relative IEMG values determined in the zone 1 using the lifting way Y, and Fig. 19 shows such relative IEMG values determined in the zone 2 using the lifting way Y. As shown in these drawings, at each of the muscles K1 to K7 shown in Fig. 15, the amount of muscle usage at the time of wearing the arm and lower back support device 10 can be confirmed to be reduced, compared to the amount of muscle usage at the time of not wearing the arm and lower back support device 10.

(Arm and lower back support device 68 according to second embodiment)

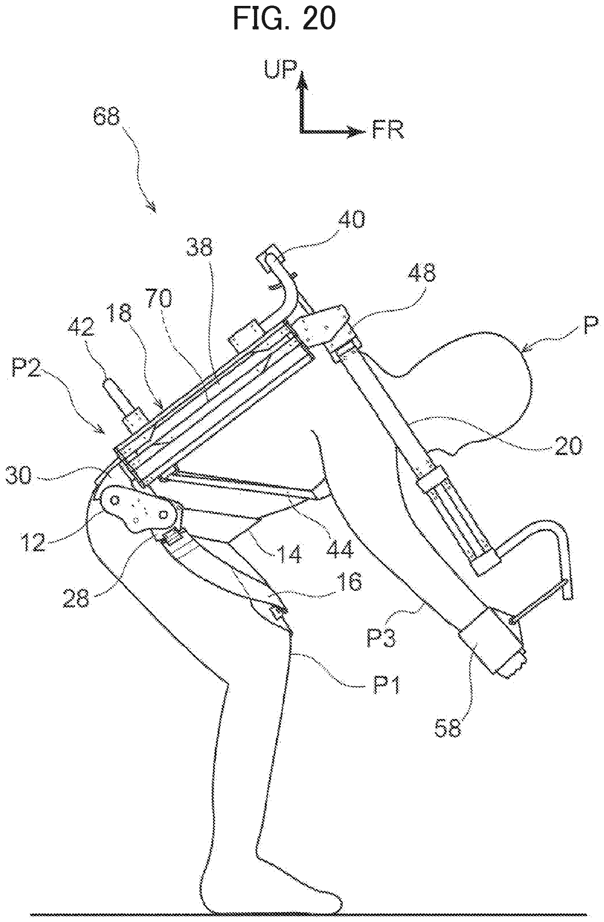

An arm and lower back support device 68 according to a second embodiment of the present invention will be described next using Figs. 20 to 23. A member and a part of each unit of the arm and lower back support device 68 of the second embodiment corresponding to those of the foregoing arm and lower back support device 10 of the first embodiment are given the same signs as the corresponding member and the corresponding part of each unit of the arm and lower back support device 10, and descriptions thereof will be omitted.

As shown in Fig. 20, the configuration of the arm and lower back support device 68 according to the second embodiment is basically the same as that of the arm and lower back support device 10 according to the first embodiment (see Fig. 1). Further, the arm and lower back support device 68 of the second embodiment is characterized in that an air spring 70 is used as the supportive force provider and as a spring instead of the artificial muscle 22.

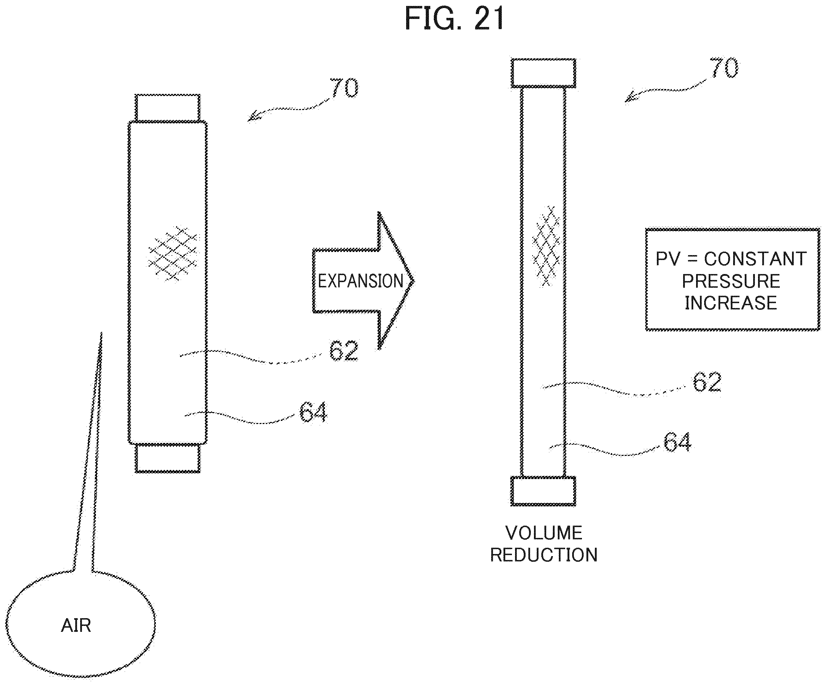

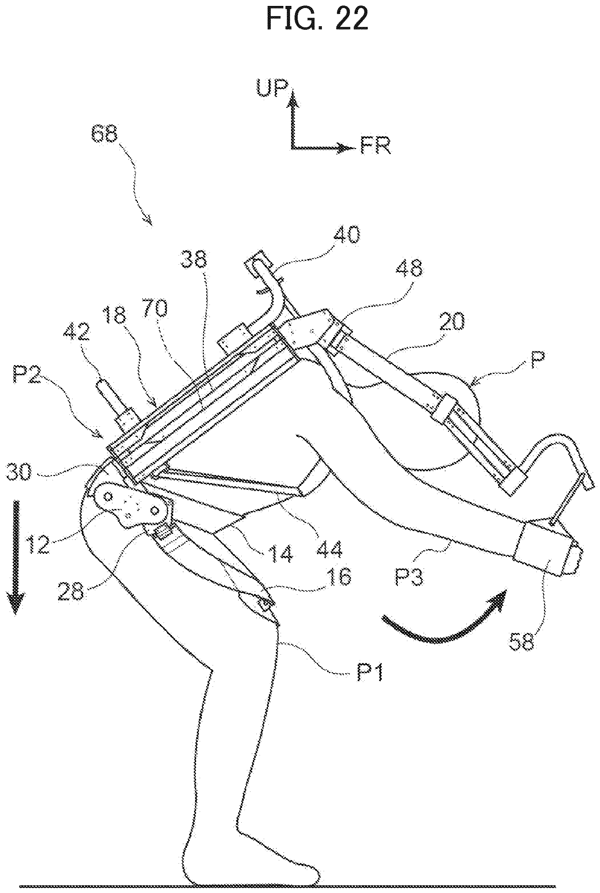

As shown in Fig. 21, the air spring 70 has a configuration formed by filling the McKibben-type artificial muscle 22 (see Fig. 6) with a certain amount of air in advance. By expanding the air spring 70, the volume of the air in the air spring 70 is reduced to increase air pressure (Charles' law). In this way, a mechanism shown in Fig. 22 is produced by which, as the posture of a user P changes to expand the air spring 70, supportive force of supporting the lower back and an arm of the user P is generated using the air spring 70.

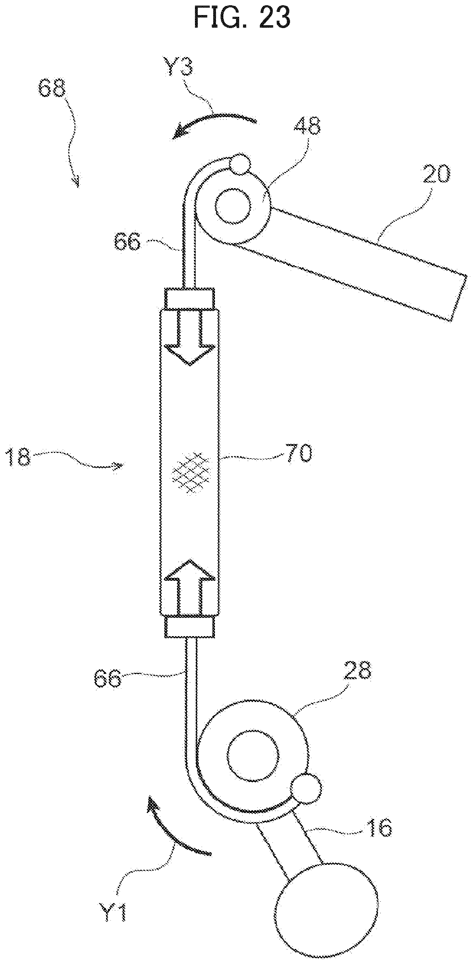

As shown in Fig. 23, in the second embodiment, the outer diameters of the first pulley 28 and the third pulley 48 (outer diameters at sections with the wound wire 66) are set at different outer diameters of 50 mm and 30 mm respectively. This causes the arm support arm 20 to tilt to a degree corresponding to about 1.67 times a change in a tilt angle of the thigh arm 16.

(Operation and effect of second embodiment)

The operation and effect of the second embodiment will be described next.

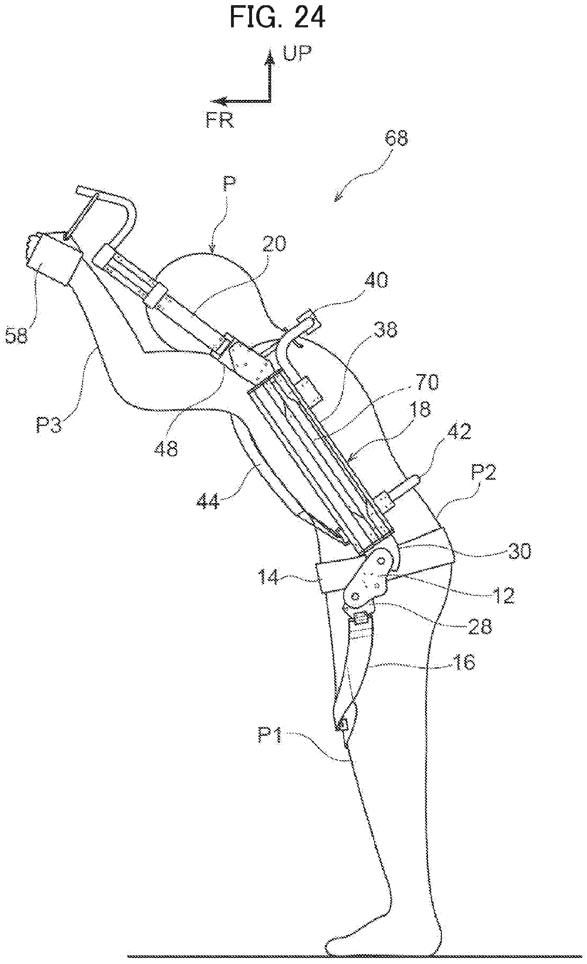

As shown in Fig. 24, the arm and lower back support device 68 of the second embodiment is mounted by the same procedure as that for mounting the foregoing arm and lower back support device 10 of the first embodiment (see Fig. 1). However, the arm and lower back support device 68 of the second embodiment is used in a different method from the foregoing arm and lower back support device 10 according to the first embodiment.

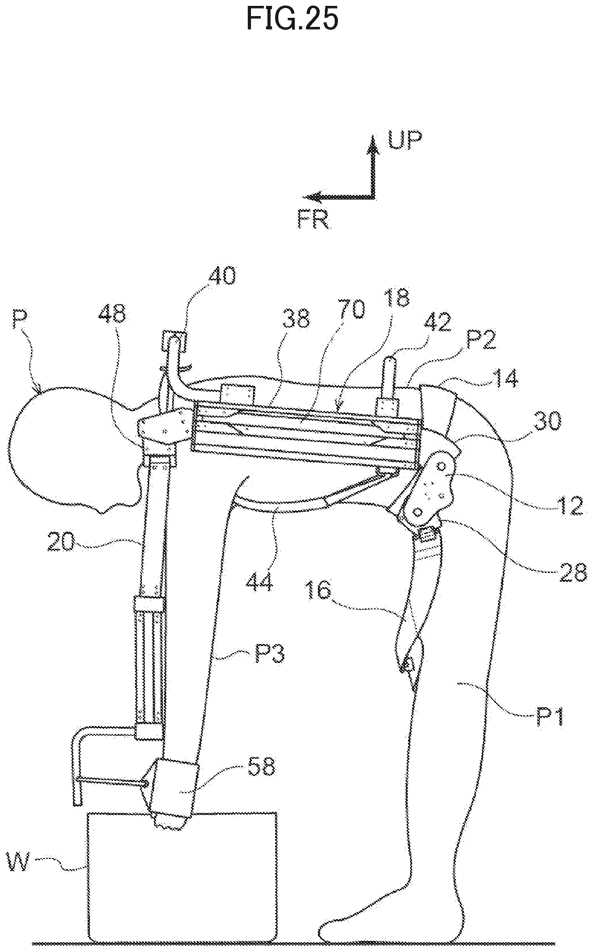

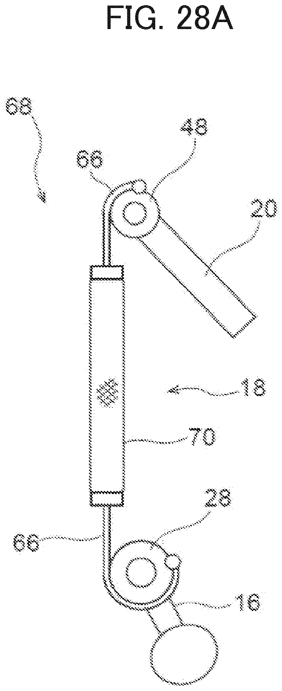

As shown in Fig. 25, according to the arm and lower back support device 68 of the second embodiment, a user P takes hold of a package W in a forward leaning posture without dropping a lower back P2. At this time, the upper body frame 18 tilts (leans forward) relative to the thigh arm 16 to expand the air spring 70 as shown in Fig. 28A.

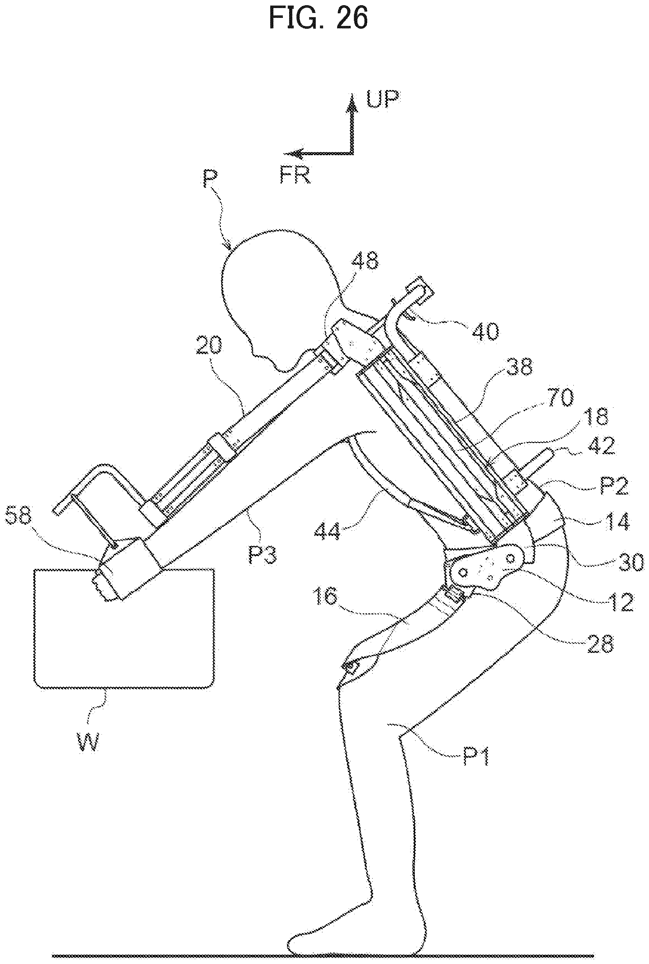

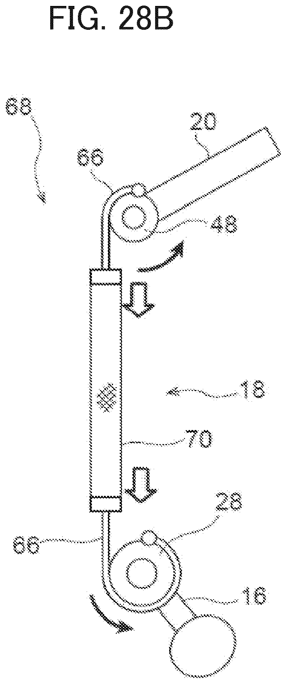

Next, as shown in Fig. 26, the user P drops the lower back P2 while bending knees P1 to increase the contractile force of the air spring 70. By doing so, the arm support arm 20 is tilted toward the upper side, as shown in Figs. 26 and 28B. As a result, when the user P lifts the package W, the lower back P2 and arms P3 of the user P are supported.

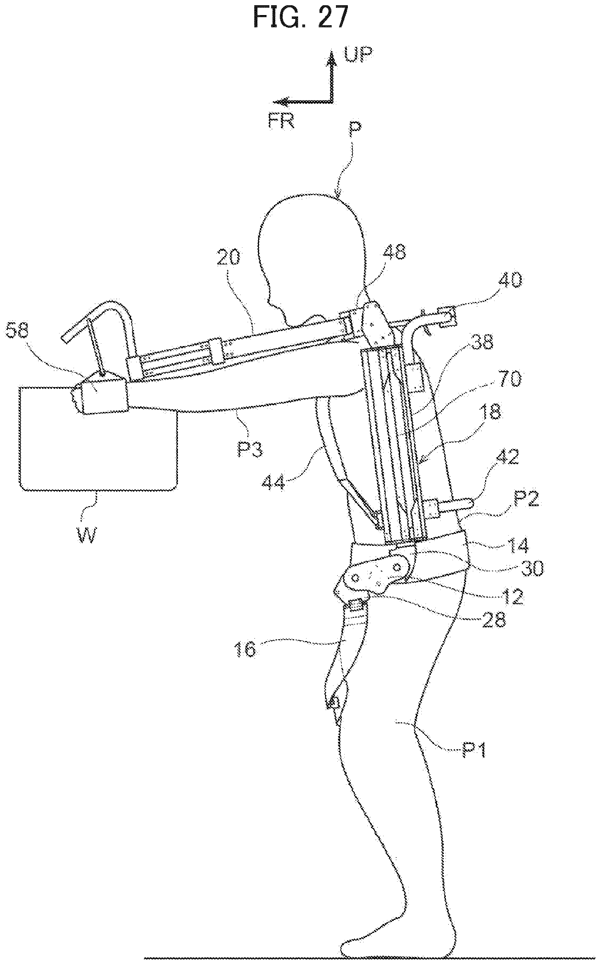

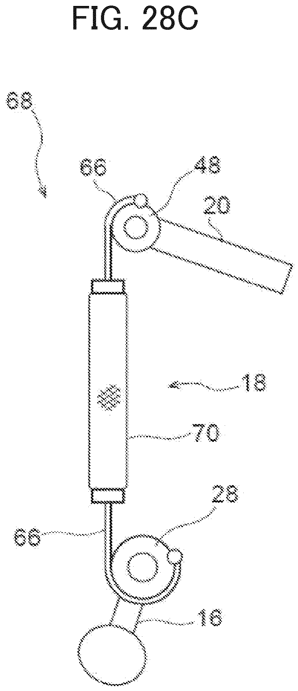

As shown in Figs. 27 and 28C, adjusting a balance between the positions of the arms P3 and that of the lower back P2 (posture) by the user P can provide support for the stretch (tilt) of the lower back P2. More specifically, the user P is allowed to acquire a ratio of distribution between supportive force on the lower back P2 and supportive force on the arms P3 as human perception (relationship in terms of support) so as to make the lower back P2 and the arms P3 movable at will. In this way, each user P is allowed to be given supportive force suitable for the motion of the user P. The second embodiment allows lifting of a package W weighing about 20 kg easily.

As described above, according to the arm and lower back support device 68 of the second embodiment, the air spring 70 generates two supportive forces including supportive force acting in a direction of raising the upper body of a user and supportive force acting in a direction of lifting an arm of the user. This makes it possible to encourage size reduction, weight reduction, and cost reduction of the arm and lower back support device 68, compared to a case where actuators are provided independently of each other for generating two supportive forces including supportive force acting in a direction of raising the upper body of a user and supportive force acting in a direction of lifting an arm of the user.

While the air spring 70 is used in the example described in the second embodiment, the air spring 70 may be replaced by a metallic coil spring, for example.

The second embodiment allows generation of supportive force in the absence of electrical or mechanical energy to be supplied from outside.

Additionally, in the second embodiment, the outer diameters of the first pulley 28 and the third pulley 48 (outer diameters at sections with the wound wire 66) are set at different outer diameters. This further encourages optimization of an angle of tilt of the arm support arm 20 relative to a change in a tilt angle of the thigh arm 16. The outer diameters of the first pulley 28 and the third pulley 48 (outer diameters at sections with the wound wire 66) can be set appropriately in consideration of a height to which a package W is to be lifted, for example.

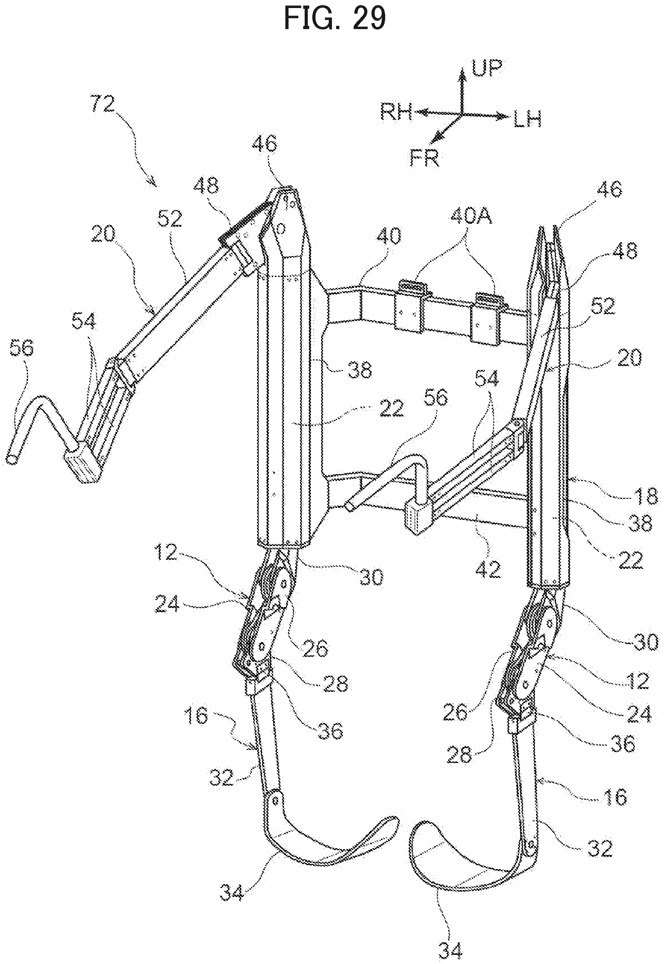

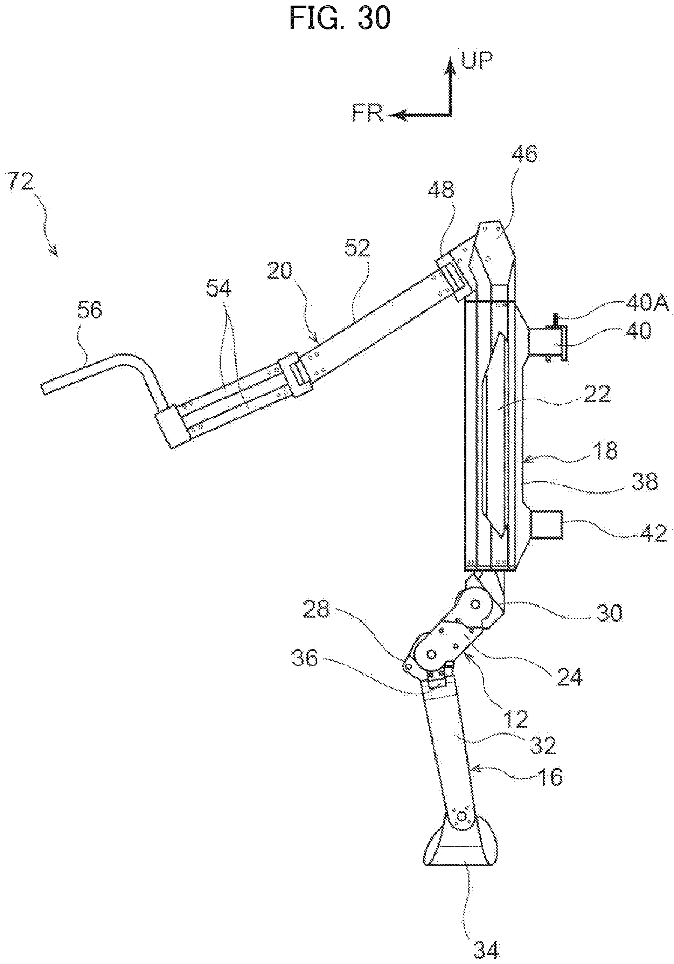

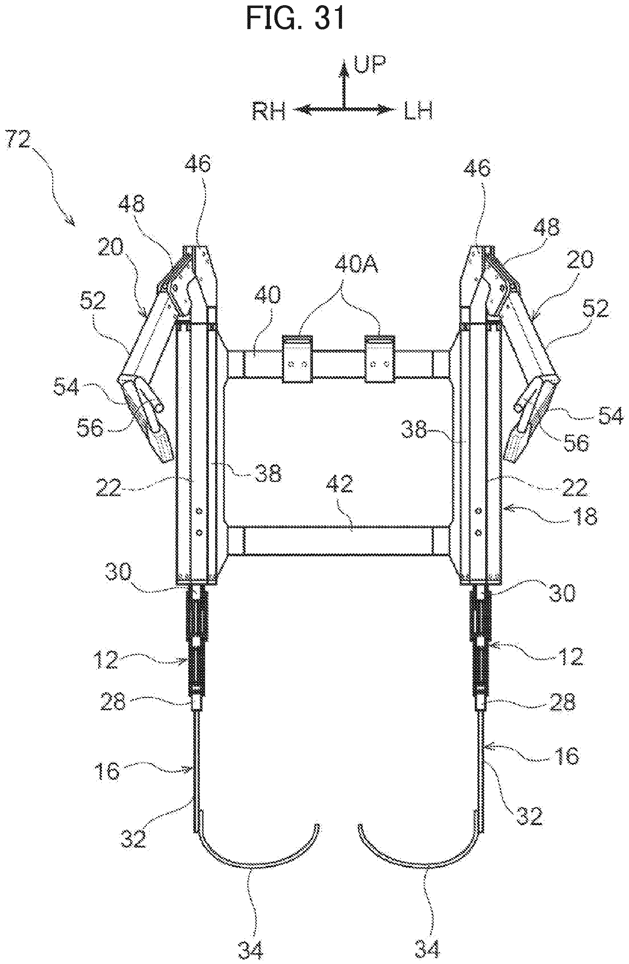

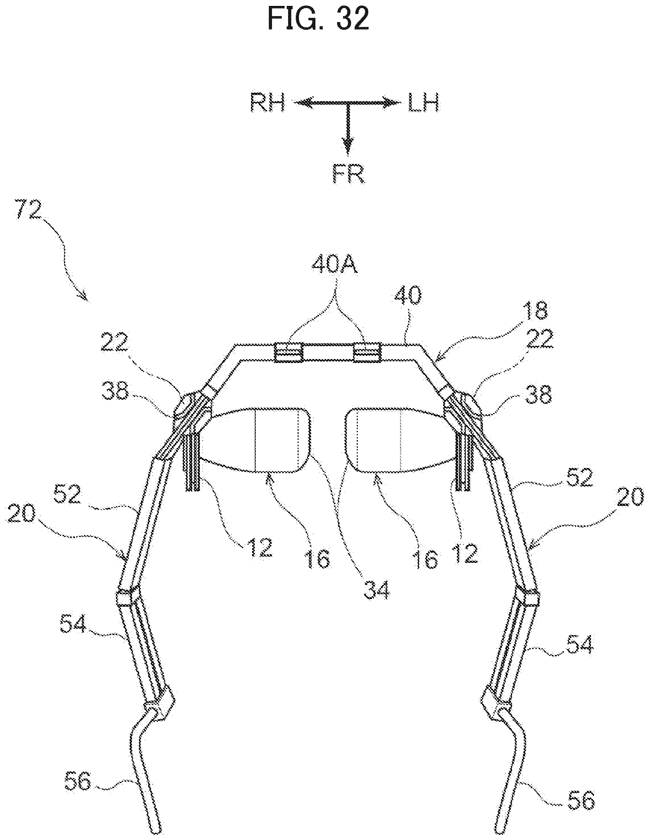

While the arm and lower back support device 10 according to the first embodiment and the arm and lower back support device 68 according to the second embodiment described above are intended to size reduction, weight reduction, and cost reduction, weight reduction and cost reduction may be encouraged further by an arm and lower back support device 72 according to a third embodiment shown in Fig. 29 to 32. In the arm and lower back support device 72 according to the third embodiment, the upper connection 40 and the lower connection 42 of the upper body frame 18 are formed using plate materials made of aluminum alloy, and the shape of the arm body 32 of the thigh arm 16 is simplified. By doing so, weight reduction and cost reduction of the arm and lower back support device 72 are encouraged further. A member and a part of each unit of the arm and lower back support device 72 corresponding to those of the foregoing arm and lower back support device 10 of the first embodiment are given the same signs as the corresponding member and the corresponding part of each unit of the arm and lower back support device 10, and descriptions thereof will be omitted.

While the one embodiment of the present invention has been described above, the present invention is not limited to the foregoing embodiment but can certainly be changed in various ways other than the above within a range not deviating from the purport of the invention.

## Claims

1. An arm and lower back support device comprising:
   a back mount to be mounted on the back of a user;
   a lower limb mount to be mounted on a lower limb of the user;
   an upper limb mount to be mounted on an upper limb of the user; and
   a supportive force provider that provides supportive force acting in a direction of raising the upper body of the user by urging the back mount toward the lower limb mount, and provides supportive force acting in a direction of lifting the upper limb of the user by urging the upper limb mount toward the back mount.

2. The arm and lower back support device according to claim 1, wherein the supportive force provider is an artificial muscle to contract in response to supply of gas, and
   the supportive force acting in the direction of raising the upper body of the user and the supportive force acting in the direction of lifting the upper limb of the user are provided by the contraction of the artificial muscle.

3. The arm and lower back support device according to claim 1, wherein the supportive force provider is a spring, and
   the supportive force acting in the direction of raising the upper body of the user and the supportive force acting in the direction of lifting the upper limb of the user are provided by the deformation of the spring caused by change in the posture of the user.

4. The arm and lower back support device according to any one of claims 1 to 3, wherein the upper limb mount is tiltable relative to the back mount in a right-left direction viewed from the user.

5. The arm and lower back support device according to any one of claims 1 to 4, wherein the upper limb mount includes a wrist mount to be mounted on a wrist of the user, and
   supportive force acting in a direction of lifting the upper limb of the user is provided from the wrist mount to the wrist of the user.

6. The arm and lower back support device according to any one of claims 1 to 5, wherein the upper limb mount is supported in a tiltable manner on the back mount through an upper limb mount side pulley,
   the lower limb mount is supported in a tiltable manner on the back mount through a lower limb mount side pulley,
   the upper limb mount side pulley and the lower limb mount side pulley have different outer diameters, and
   a wire connected to the supportive force provider is engaged with each of the upper limb mount side pulley and the lower limb mount side pulley.

7. An arm and lower back support device comprising:
a back mount to be mounted on the back of a user;
a lower limb mount to be mounted on a lower limb of the user;
an upper limb mount to be mounted on an upper limb of the user; and
a supportive force provider that provides supportive force acting in a direction of raising the upper body of the user by urging the back mount toward the lower limb mount, and provides supportive force acting in a direction of lifting the upper limb of the user by urging the upper limb mount toward the back mount, wherein
a ratio of distribution between the supportive force acting in the direction of lifting the upper limb of the user and the supportive force acting in the direction of raising the upper body of the user changes in response to motion of the user.

# FIG. 1

# FIG. 2

# FIG. 3

# FIG. 4

# FIG. 5

58

58A

FIG. 6

# FIG. 7

# FIG. 8

## FIG. 9

# FIG. 10

# FIG. 11

# FIG. 12

MOTION ZONE

$$EMG [mV]$$

Time [sec]

**ELECTROMYOGRAM**

→ AVERAGE RECTIFIED VALUE →

MOTION ZONE

$$ARV [mV]$$

Time [sec]

**ARV**

→ INTEGRATION →

EVALUATION VALUE

**IEMG**

AMOUNT OF MUSCLE USAGE : HIGH

⇩

IEMG : HIGH

EP 3 620 274 A1

# FIG. 13A

UP

FR

P2

P

P1

P3

W

# FIG. 13B

# FIG. 13C

# FIG. 14A

# FIG. 14B

# FIG. 14C

# FIG. 15

FIG. 16

EP 3 620 274 A1

FIG. 17

FIG. 18

EP 3 620 274 A1

FIG. 19

EP 3 620 274 A1

# FIG. 20

# FIG. 21

70

70

EXPANSION

PV = CONSTANT
PRESSURE
INCREASE

62

64

62

64

VOLUME
REDUCTION

AIR

EP 3 620 274 A1

# FIG. 22

# FIG. 23

# FIG. 24

# FIG.25

# FIG. 26

# FIG. 27

# FIG. 28A

# FIG. 28B

# FIG. 28C

# FIG. 29

# FIG. 30

# FIG. 31

# FIG. 32

<div align="center">

**INTERNATIONAL SEARCH REPORT**

</div>

| | International application No. |
|---|---|
| | PCT/IB2018/054890 |

**A. CLASSIFICATION OF SUBJECT MATTER**
Int.Cl. B25J11/00(2006.01)i, A61F2/54(2006.01)i

According to International Patent Classification (IPC) or to both national classification and IPC

**B. FIELDS SEARCHED**

Minimum documentation searched (classification system followed by classification symbols)
Int.Cl. B25J11/00, A61F2/54

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | 須賀裕文，肉体労働者支援用マッスルスーツの開発，第３０回日本ロボット学会学術講演会予稿集 DVD-ROM 2012 年 The 30th Annual Conference of the Robotics Society of Japan, 17 September 2012, page 1, left column, line 21 to right column, line 26, page 2, right column, lines 7-22, fig. 1-4, 11-12, non-official translation (SUGA, Hirofumi, "Development of muscle suit for supporting manual laborers") | 1-2, 4-5, 7<br>3, 6 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 14 August 2018 (14.08.2018) | 21 August 2018 (21.08.2018) |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**INTERNATIONAL SEARCH REPORT**

| International application No. |
|---|
| PCT/IB2018/054890 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2016-10451 A (SHARP CORP.) 21 January 2016, paragraphs [0138]-[0142], fig. 1 (Family: none) | 3 |
| Y | JP 2012-239818 A (TOKYO UNIVERSITY OF SCIENCE) 10 December 2012, paragraphs [0035], [0040], [0047], [0052], fig. 2 (Family: none) | 6 |
| A | JP 2013-138848 A (WAKAYAMA UNIV.) 18 July 2013, paragraphs [0010]-[0011], [0048]-[0057], fig. 1 & US 2014/0212243 A1, paragraphs [0015]-[0017], [0116]-[0124], fig. 1A-1B & WO 2013/035814 A1 & EP 2754538 A1 | 1-7 |
| A | JP 2015-182832 A (KUBOTA CORP.) 22 October 2015, paragraphs [0023]-[0029], [0043]-[0052], [0060]-[0061], [0066]-[0067], fig. 1-2 (Family: none) | 1-7 |

Form PCT/ISA/210 (continuation of second sheet) (January 2015)

**EP 3 620 274 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009273711 A

- JP 2016002123 A